# EUROPEAN PATENT SPECIFICATION

(11) **EP 1 748 068 B1**
(45) Date of publication and mention of the grant of the patent: **03.03.2010**
(21) Application number: 05727395.5
(22) Date of filing: 28.03.2005
(51) Int. Cl.: C12N 15/09, C12N 15/88, C12N 1/15, C12P 7/64

(54) **METHOD OF BREEDING LIPID-PRODUCING STRAIN AND UTILIZATION OF THE SAME**
VERFAHREN ZUR ZÜCHTUNG EINES LIPIDPRODUZIERENDEN STAMMS UND NUTZUNG DAVON
PROCÉDÉ D'ÉLEVAGE D'UNE SOUCHE PRODUISANT DES LIPIDES ET UTILISATION DE CELUI-CI

(30) Priority: 31.03.2004 JP 2004107512
(43) Date of publication of application: 31.01.2007
(73) Proprietor: Suntory Holdings Limited, Kita-ku, Osaka-shi, Osaka 530-8203 (JP)
(72) Inventor: OCHIAI, Misa, Mishima-gun, Osaka 618-0001 (JP); KAWASHIMA, Hiroshi, Takatsuki-shi, Osaka 569-1121 (JP); SHIMIZU, Sakayu, Ukyo-ku, Kyoto-shi, Kyoto 616-8212 (JP); SAKURADANI, Eiji, Muko-shi, Kyoto 617-0006 (JP)
(74) Representative: Vossius & Partner
(86) International application number: PCT/JP2005/005786
(87) International publication number: WO 2005/095597

(56) References cited:
- EP-A2- 0 825 263
- CERTIK MILAN ET AL: "Desaturase-defective fungal mutants: Useful tools for the regulation and overproduction of polyunsaturated fatty acids" TRENDS IN BIOTECHNOLOGY, vol. 16, no. 12, December 1998 (1998-12), pages 500-505, XP004143810 ISSN: 0167-7799
- UEDA R: "RNAI: A NEW TECHNOLOGY IN THE POST-GENOMIC SEQUENCING ERA" JOURNAL OF NEUROGENETICS, ELSEVIER, AMSTERDAM, NL, vol. 15, no. 3/4, 2001, pages 193-204, XP001147227 ISSN: 0167-7063
- NICOLAS F E ET AL: "Two classes of small antisense RNAs in fungal RNA silencing triggered by non-integrative transgenes" EMBO JOURNAL, OXFORD UNIVERSITY PRESS, SURREY, GB, vol. 22, no. 15, 1 August 2003 (2003-08-01), pages 3983-3991, XP002282396 ISSN: 0261-4189
- MACKENZIE D A ET AL: "Isolation and use of a homologous histone H4 promoter and a ribosomal DNA region in a transformation vector for the oil-producing fungus Mortierella alpina" APPLIED AND ENVIRONMENTAL MICROBIOLOGY, WASHINGTON,DC, US, vol. 66, no. 11, November 2000 (2000-11), pages 4655-4661, XP002291019 ISSN: 0099-2240
- TAKENO SEIKI ET AL: "Improvement of the fatty acid composition of an oil-producing filamentous fungus, Mortierella alpina 1S-4, through RNA interference with Delta 12-desaturase gene expression" APPLIED AND ENVIRONMENTAL MICROBIOLOGY, vol. 71, no. 9, September 2005 (2005-09), pages 5124-5128, XP002437136 ISSN: 0099-2240
- SHIMIZU S. ET AL.: 'Mortierella alpine IS-4 Oyobi Sono Hen'i Kabu i yoru Kado Fuhowa Shibosan Gan'yu Yushi no Seisan.' YUKAGAKU vol. 42, no. 4, 1993, pages 254 - 264, XP002992659
- PARKER-BARNES J. ET AL.: 'Identification and caracterization of an ezyme involved in the elongation of n-6 and n-3 polyunsaturated fatty acids.' PROC.NATL.ACAD.SCI.USA vol. 97, 2000, pages 8284 - 8289, XP002992660
- KADOTANI N. ET AL.: 'RNA Silencing in the phytopathogenic fungus Magnaporthe oryzae.' MOL. PLANT.MICROBE INTERACT. vol. 16, no. 9, 2003, pages 769 - 776, XP009036113

### SEQUENCE LISTING

<110> SUNTORY LIMITED
<120> CULTIVATION METHOD OF LIPID PRODUCING FUNGI AND USE OF SUCH A METHOD
<130> P160307
<150> JP 2004-107512
   <151> 2004-3-31
<160> 18
<170> PatentIn Ver. 2.1
<210> 1
   <211> 30
   <212> DNA
   <213> Artificial Sequence
<220>
   <223> Description of Artificial Sequence: HisProFX
<400> 1
   tacgaattca agcgaaagag agattatgaa 30
<210> 2
   <211> 30
   <212> DNA
   <213> Artificial Sequence
<220>
   <223> Description of Artificial Sequence: TrpCRX
<400> 2
   gaagaattcc ctctaaacaa gtgtacctgt 30
<210> 3
   <211> 31
   <212> DNA
   <213> Artificial Sequence
<220>
   <223> Description of Artificial Sequence: MAELORNAil-1
<400> 3
   ctggatccta tggccgccgc aatcttggac a 31
<210> 4
   <211> 30
   <212> DNA
   <213> Artificial Sequence
<220>
   <223> Description of Artificial Sequence: MAELORNAi3
<400> 4
   aaccatggtc atccctaggt ggaagtaatg 30
<210> 5
   <211> 30
   <212> DNA
   <213> Artificial Sequence
<220>
   <223> Description of Artificial Sequence: MAELORNAi1
<400> 5
   ttggatccat ggccgccgca atcttggaca 30
<210> 6
   <211> 30
   <212> DNA
   <213> Artificial Sequence
<220>
   <223> Description of Artificial Sequence: MAELORNAi5
<400> 6
   tgatctccta ggtggaacac tgatagccac 30
<210> 7
   <211> 20
   <212> DNA
   <213> Artificial Sequence
<220>
   <223> Description of Artificial Sequence: RDNA1
<400> 7
   acaggtacac ttgtttagag 20
<210> 8
   <211> 20
   <212> DNA
   <213> Artificial Sequence
<220>
   <223> Description of Artificial Sequence: RDNA2
<400> 8
   cgctgcgttc ttcatcgatg 20
<210> 9
   <211> 25
   <212> DNA
   <213> Artificial Sequence
<220>
   <223> Description of Artificial Sequence: MAELO-1
<400> 9
   agtccatcga ctccttcgtc ttcca 25
<210> 10
   <211> 25
   <212> DNA
   <213> Artificial Sequence
<220>
   <223> Description of Artificial Sequence: MAELO-2
<400> 10
   cggtgtcagc caactcccag tactt 25
<210> 11
   <211> 27
   <212> DNA
   <213> Artificial Sequence
<220>
   <223> Description of Artificial Sequence: d12-1
<400> 11
   gcggatccat ggcacctccc aacacta 27
<210> 12
   <211> 28
   <212> DNA
   <213> Artificial Sequence
<220>
   <223> Description of Artificial Sequence: d12-2
<400> 12
   agaggccttc ataataaggt acgcaggc 28
<210> 13
   <211> 25
   <212> DNA
   <213> Artificial Sequence
<220>
   <223> Description of Artificial Sequence: d12-3
<400> 13
   ttgctattga tctgacctgg gcctc 25
<210> 14
   <211> 24
   <212> DNA
   <213> Artificial Sequence
<220>
   <223> Description of Artificial Sequence: d12-4
<400> 14
   tgggaacaaa gacctggtcc ttgg 24
<210> 15
   <211> 29
   <212> DNA
   <213> Artificial Sequence
<220>
   <223> Description of Artificial Sequence: Primer MAELO-H
<400> 15
   gcaagcttat ggccgccgca atcttggac 29
<210> 16
   <211> 30
   <212> DNA
   <213> Artificial Sequence
<220>
   <223> Description of Artificial Sequence: Primer MAELO-S1
<400> 16
   gcactagttt agatgtgctt gctgttggag 30
<210> 17
   <211> 30
   <212> DNA
   <213> Artificial Sequence
<220>
   <223> Description of Artificial Sequence: Primer MAELORNAi3
<400> 17
   tgccatgggg aaatatgccc taggccatgc 30
<210> 18
   <211> 27
   <212> DNA
   <213> Artificial Sequence
<220>
   <223> Description of Artificial Sequence: Primer MAELORNAi4
<400> 18
   cacttaccta ggggcttctt cttgagg 27

## Description

### TECHNICAL FIELD

The present invention relates to a breeding method of lipid producing fungi and use of such a method. The invention particularly relates to a method for the production of lipid producing fungi that belong to genus *Mortierella,* said method comprising an expression suppressing step of suppressing expression of a lipid metabolism gene encoding GLELO, MAELO, Δ5 fatty acid desaturase, Δ6 fatty acid desaturase or Δ12 fatty acid desaturase in the lipid producing fungi, wherein said expression suppressing step includes an RNAi step of suppressing expression of the lipid metabolism gene by an RNAi method or a cosuppression step of suppressing expression of the lipid metabolism gene by a cosuppression method.

### BACKGROUND ART

There have been ongoing developments and actual applications of techniques for producing useful compounds through metabolism of microorganisms (broadly, fermentation techniques). In one specific example, lipid producing fungi are known that have the ability to produce a large amount of lipids through metabolism. Representative examples of such lipid producing fungi include *Mortierella alpina* and other species of genus *Mortierella.* The *Mortierella* are known to produce arachidonic acid and other polyunsaturated fatty acids (PUFA), and for this reason *Mortierella* are highly useful in industrial applications (see Patent Document 1, for example).

In a variety of useful organisms including microorganisms such as the lipid producing fungi, it is common practice to breed the organisms, i.e., modify the genetic characteristics of the organisms to achieve more desirable characteristics (breed improvement). This is particularly important in fermentation techniques, in which efficient production of compounds by the microorganisms is sought for to reduce manufacturing cost.

Breeding is basically a two-step process. In the first step, a population with genetic variations is prepared (simply "population preparing step"). In the second step, a breed or strain with desirable characteristics is screened for (simply "screening step"). The population preparing step and screening step can be carried out in a variety of ways depending on the type of microorganism used. In the case of microorganisms such as the lipid producing fungi, (1) mutation or (2) transformation is generally employed in the population preparing step.

### (1) Method employing mutation

In a population preparing step employing mutation, a population of microorganisms is prepared by introducing mutation in a variety of ways. However, the mutation occurs randomly and many different types of mutations result. As such, while it may be possible to obtain a breed (strain) with a target trait in the screening step, there is always a possibility that unexpected damage is caused on genes other than the genes associated with the target trait. For example, in the case of the lipid producing fungi, a change in the type of produced lipid may accompany reduced proliferating ability or spore forming ability, among other things. Therefore, it is not necessarily the case that a strain with good productivity is obtained in the population preparing step employing mutation.

Further, in the method employing mutation, many different kinds of mutations randomly occur in the individuals making up the population. Thus, if a suitable screening method is not available, it takes a tremendous effort to find a mutant (strain) with a target trait because, in this case, all individuals of the population need to be screened for each different kind of mutation.

### (2) Method employing transformation

In a population preparing step employing transformation, a population of transformants is obtained by introducing DNA fragments required for acquiring a target trait (transformation), using the bred organism as a host. That is, expression of only target-specific genes is controlled in the population. As such, in the next screening step, it is only required to screen for a desirable breed (strain) from the transformants. Screening is therefore easier, and other genes are prevented from unexpected damages. As a result, the labor required for the breeding can be significantly reduced.

As described above, the population preparing step of breeding is more desirably carried out by way of transformation. For example, there have been many reports concerning transformation methods for filamentous fungi including genus *Mortierella.*

Specifically, (a) Non-Patent Documents 1 through 3 and other publications disclose techniques for transforming filamentous fungi such as *Aspergillus nidulans* or *Neurospora crassa* by a particle delivery method. In these techniques, a uracil auxotrophic strain is used as a transformed host strain, and a transformed strain is screened for by using the complementary gene as a marker gene.

As to a transformation method of *M. alpina,* a technique disclosed in Non-Patent Document 4 has been known. In this technique, the spore is turned into a protoplast, and genes are introduced into the cell by an electroporation method. For the screening of transformants, hygromycin B resistance gene (hpt) from *E. coli* is used as a marker gene, and transformants that can grow in a hygromycin-containing medium are screened for.

In modifying genetic characteristics of useful organisms to more desirable characteristics, there are cases where functions of specific genes are intentionally removed either partially or completely. While this can be achieved by the method employing mutation as described in section (1) above, the method poses some disadvantages as noted above. Therefore, there is a need for a technique of breeding mutant strains with the method employing transformation as described in section (2) above, so that functions of specific genes can be partially or completely removed both easily and reliably.

In one known technique of obtaining such a mutant strain with the partial or complete functional removal of specific genes, expression of specific genes is suppressed. Some of the examples of such a technique include an RNAi method, a gene deletion method in which genes on the chromosome are deleted by homologous recombination, a co-suppression method, and an antisense method. Among these methods, the method employing homologous recombination is known to provide stable results. The RNAi method, which is fairly new, has been reported in many organisms, and is known to be highly effective.
[Patent Document
   Japanese Examined Patent Publication No. 34752/1995 (Tokukouhei 7-34752, published on April 19, 1995), Japanese Laid-Open Patent Publication No. 44891/1988 (Tokukaisho 63-44891, published on February 25, 1988)
[Non-Patent Document 1]
   Fungaro M. H. et al. Fems microbial Lett., 25, 293-297, 1995
[Non-Patent Document 2]
   Herzog R. W. et al. Appl. Microbiol. Biotechnol., 45, 333-337, 1996
[Non-Patent Document 3]
   Armaleo, D. et al. Curr. Genet., 17, 97-103, 1990
[Non-Patent Document 4]
   Mackenzie D. A. et al. Appl. Environ. Microbiol., 66, 4655-4661, 2000

### DISCLOSURE OF INVENTION

However, there has been no report as to a method of suppressing expression of specific genes in lipid producing *Mortierella.*

Further, while the gene expression repressing effects of the RNAi method have been well-documented in many organisms for example, whether or not the method will be effective in a specific organism cannot be known until the method is actually carried out. For example, there has been no report that suggests the effectiveness of the RNAi method in lipid producing *Mortierella.*

Many PUFAs are essential fatty acids, and are involved in complex physiological functions in the body. Thus, the importance of PUFA as an important nutrient has won the recognition in the last years. This has called for a fermentation technique that allows for more efficient PUFA production. However, such a fermentation technique cannot be achieved without a technique of efficiently and effectively breeding *Mortierella,* which are known to produce lipids reliably.

The present invention was made in view of the foregoing problems, and an object of the invention is to provide a breeding method for effectively and efficiently breeding lipid producing *Mortierella* by suppressing expression of specific genes. The invention also provides use of such a method.

In accomplishing the invention, the inventors resorted to the RNAi method to obtain a *Mortierella* mutant strain in which expression of MAELO gene or Δ12 fatty acid desaturase gene was suppressed. A functional analysis of the mutant strain revealed that the mutant strain produced very long-chain saturated fatty acids in reduced proportions in the case of the MAELO gene, and that the mutant strain produced mead acid in the case of the Δ12 fatty acid desaturase gene.

Specifically, the invention provides industrially useful method or substances as defined in (1) through (5)below.
(1) A method for the production of lipid producing fungi that belong to genus *Mortierella,* said method comprising an expression suppressing step of suppressing expression of a lipid metabolism gene encoding GLELO, MAELO, Δ5 fatty acid desaturase, Δ6 fatty acid desaturase or Δ12 fatty acid desaturase in the lipid producing fungi, wherein said expression suppressing step includes an RNAi step of suppressing expression of the lipid metabolism gene by an RNAi method or a cosuppression step of suppressing expression of the lipid metabolism gene by a cosuppression method.
(2) The method of item 1, wherein said RNAi step includes a transformation step of introducing a recombinant expression vector into the lipid producing fungi, wherein the recombinant expression vector causes expression of double stranded RNA corresponding to all of or part of a nucleotide sequence of the lipid metabolism gene.
(3) The method of item 2, wherein said RNAi step further includes an expression vector constructing step of constructing the recombinant expression vector.
(4) The method of item 2 or 3, wherein the transformation step is carried out by an electroporation method or a particle delivery method.
(5) The method of any one of items 1 to 4, wherein the lipid producing fungi are *Mortierella alpina.*

### BEST MODE FOR CARRYING OUT THE INVENTION

The present invention provides a method the production of lipid producing fungi that belong to genus *Mortierella,* said method comprising an expression suppressing step of suppressing expression of a lipid metabolism gene encoding GLELO, MAELO, Δ5 fatty acid desaturase, Δ6 fatty acid desaturase or Δ12 fatty acid desaturase in the lipid producing fungi, wherein said expression suppressing step includes an RNAi step of suppressing expression of the lipid metabolism gene by an RNAi method or a cosuppression step of suppressing expression of the lipid metabolism gene by a cosuppression method.

The following will describe *Mortierella,* a breeding method, and use of the method in this order, according to the present invention.

### [1] Mortierella

The *Mortierella* used in a breeding method according to the present invention are not particularly limited, and various kinds of filamentous fungi of genus *Mortierella* can be used. Genus *Mortierella* include two subgenera, *Mortierella* and *Micromucor*. The fungi that belong to subgenus *Mortierella* all produce fatty acids with 20 carbon atoms, such as arachidonic acid, whereas those belonging to subgenus *Micromucor* produce fatty acids with 18 or less carbon atoms. The *Mortierella* used in the present invention may belong to either *Mortierella* or *Micromucor*. Specifically, non-limiting examples of *Mortierella* include: *M. alpina, M. elongata, M. exigua, M. hygrophila, M. isabellina, M. turficola, M. gamsii, M. cogitans, M. capitata, and M. vinacea.* Among these examples, *Mortierella alpina*, common lipid producing fungi, is particularly preferable in the present invention. Some strains of *M. alpina* are known to store arachidonic acid (ARA) and other PUFAs. For this reason, *M. alpina* has been widely used not only for the research of PUFA biosynthesis but also in industries producing PUFA.

The source of *M. alpina* or other *Mortierella* are not particularly limited. For example, the fungi can be obtained from various microorganism depositary institutions such as the Institute for Fermentation or ATCC (American Type Culture Collection). In the case of strains for which patent applications have been filed, the organisms can be obtained from the International Patent Organism Depositary in the National Institute of Advanced Industrial Science and Technology. Alternatively, an unknown strain of *Mortierella* may be obtained from natural environment by a known screening method.

### [2] Breeding method of lipid producing fungi according to the present invention

A breeding method according to the present invention includes an expression suppressing step by a co-suppression method or an RNAi method. Among these methods, the RNAi method is particularly preferable since it is simple and yields good results, for example.

That is, it is preferable that the expression suppressing step include an RNAi step of suppressing expression of the above-mentioned lipid metabolism genes. More specifically, DNA fragments that cause RNAi are introduced into lipid producing *Mortierella.* With the resulting expression of double stranded RNA, expression of the lipid metabolism genes is suppressed and therefore lipid producing fungi with desirable characteristics are bred.

It is preferable that the RNAi step include a transformation step and an expression vector constructing step. It is to be noted that a breeding method of lipid producing fungi according to the present invention may include additional steps as well. The following will describe the respective steps of the expression suppressing step in detail.

### [2-1] RNAi step

The RNAi step according to the present invention is not particularly limited as long as expression of the lipid metabolism gene in lipid producing fungi is suppressed by an RNAi method. As such, the step is not just limited to particular methods, conditions, or materials.

As used herein, the "RNAi method" refers to a method in which gene expression is suppressed by a phenomenon known as RNAi. The "RNAi" refers to the situation where the presence of double stranded RNA ("dsRNA") in the cell promotes degradation of mRNA that hybridizes with the dsRNA, and thereby suppresses expression of genes corresponding to the mRNA.

Therefore, the RNAi step can be regarded as a step of introducing dsRNA complementary to the nucleotide sequence that codes for the entire part or some part of a gene whose expression is to be suppressed. The nucleotide sequence or length of introduced RNA is not particularly limited as long as it can suppress expression of a target gene. For this purpose, techniques of conventional RNAi methods can be suitably used.

The method of introducing RNA into the lipid producing fungi is not particularly limited either, and can be carried out according to conventional RNAi methods. In one specific example of introducing dsRNA into the cell, plasmid DNA (recombinant expression vector) that codes for RNAi-inducing RNA is introduced into the cell, and is expressed in the cell to suppress gene expression, as will be described later in Examples. Other examples include a liposome method, an electroporation method, and a microinjection method. That is, the RNAi method preferably includes an expression vector constructing step and a transformation step, as described below.

### [2-1-1] Expression vector constructing step

The expression vector constructing step according to the present invention is a step of constructing a recombinant expression vector in *Mortierella* (lipid producing fungi), wherein the recombinant expression vector is used to express double stranded RNA corresponding to the entire part or some part of the nucleotide sequence of a predetermined gene whose expression should be suppressed. That is, the step is not particularly limited as long as a recombinant expression vector is constructed in *Mortierella* such that the recombinant expression vector, with a suitable sequence of DNA ligated under the control of a promoter, brings about expression of double stranded RNA that causes RNAi. In other words, in the expression vector constructing step, a recombinant expression vector is constructed in such a manner that a gene that encodes the double stranded RNA is expressed under the control of a promoter.

The gene whose expression should be suppressed is a gene encoding GLELO, MAELO, Δ5 fatty acid desaturase, Δ6 fatty acid desaturase or Δ12 fatty acid desaturase.

The method of constructing a recombinant expression vector for RNAi is not particularly limited, and conventional methods can be used therefor. For example, gene cassettes ligated via a linker sequence or other sequences are transcribed *in vivo* in such a manner that both the sense strand and antisense strand of a gene whose expression should be suppressed are transcribed, so as to generate RNA (inverted repeat RNA) that forms a double strand within the molecule. In this manner, expression of a host gene corresponding to the RNA can be suppressed. The method enables RNAi to be continuously generated in the cell, and therefore suppresses expression of the target gene over an extended time period. Note that, the provision of a linker sequence is optional but preferable.

In one specific example of the expression vector constructing step, reverse cDNA of the target gene, a linker sequence, and forward cDNA of the target gene are ligated in this order and inserted downstream of a promoter that can be expressed in the host cell, so as to prepare a construct (RNAi plasmid). The RNAi plasmid is then introduced into lipid producing fungi and transcribed *in vivo*, so as to generate inverted repeat RNA that forms a double strand within the molecule.

Note that, the order or direction of the antisense strand and sense strand incorporated in the RNAi plasmid is not particularly limited as long as it allows the RNA (inverted repeat RNA) generated by transcription *in vivo* in the host to form double stranded RNA within the molecule.

Further, the length of the nucleotide sequence of the double stranded RNA is not particularly limited as long as it can efficiently cause RNAi. Further, in the case of double stranded RNA corresponding to only some part of the gene, a portion of the gene is suitably selected depending on purpose.

The promoter is not particularly limited either as long as it can effectively express the gene that encodes the double stranded RNA in *Mortierella* (lipid producing fungi). As such, conventional promoters can be suitably used. A non-limiting example of such conventional promoters is hisH4.1 promoter.

Various types of conventional vectors can be used to provide the recombinant expression vector. Some of the examples include a plasmid, phage, and cosmid, which are suitably selected according to the type of host cell (type of *Mortierella*) or the employed method of introduction. Specific examples include pBR322, pBR325, pUC-type, pBluescript-type, and pBI-type vectors.

The recombinant expression vector may include DNA segments other than the promoter and the gene encoding the double stranded RNA. Non-limiting examples of such DNA segments include a terminator, a selection marker, an enhancer, and a nucleotide sequence for improving translation efficiency.

The type of terminator is not particularly limited as long as it serves as a termination site of transcription. As such, conventional terminators can be used. The terminator is placed at a suitable position in the recombinant expression vector, so as to prevent synthesis of an excessively long transcript in the vector-introduced cell. A non-limiting example of the terminator is the trpC terminator used in the invention.

The selection marker may be, for example, a gene that complements auxotrophy, or a drug-resistant gene. Specific examples of genes that complement auxotrophy include: genes that complement auxotrophy for amino acid such as leucine, histidine, methionine, arginine, tryptophan, or lysine; a gene that complements auxotrophy for nucleic acid such as uracil or adenine; and a gene that complements auxotrophy for vitamins. In this way, a medium depleted with a specific nutrient such as above (medium without specific nutrients) can be easily screened for transformed strains that have incorporated the recombinant expression vector. Specific examples of drug-resistant genes include genes resistant to ampicillin, hygromycin, bleomycin, kanamycin, gentamicin, or chloramphenicol. In this way, transformed strains that have incorporated the recombinant expression vector can be selected by simply screening an antibiotic-containing medium for the strains that grow in the medium.

The enhancer is not particularly limited as long as it is a DNA nucleotide sequence that promotes transcription of an adjacent gene on a single DNA strand, i.e., in a cis position. For example, a repeating sequence of 72 base long near the replication origin of monkey-derived simian virus 40 (SV 40) can be used as an enhancer. Other conventional enhancers can be used as well. With the use of an enhancer, the transcription activity can be improved even when the promoter region alone is insufficient to obtain a sufficient level of desired gene expression. As described above, various types of DNA segments can be included in the recombinant expression vector depending on intended use of the vector or the type of vector-introduced cell.

The method of constructing the recombinant expression vector is not particularly limited either. Generally, the promoter, the gene encoding double stranded RNA, and optionally other DNA segments are introduced in a predetermined order into a suitably selected carrier. For example, the gene encoding double stranded RNA and the promoter (optionally, an enhancer, terminator, and the like) are ligated to one another to construct an expression cassette, which is then introduced into the vector.

In the construction of the expression cassette, the order of DNA segments can be specified by providing each DNA segment with complementary staggered ends, and by joining the segments with a ligase. When the expression cassette includes a terminator, the terminator is positioned downstream of the promoter and the gene encoding double stranded RNA in this order. When the expression cassette includes an enhancer, the enhancer is positioned downstream of the promoter and upstream of the gene encoding double stranded RNA. The type of reagent, such as the restriction enzyme or ligase, used to construct the recombinant expression vector is not particularly limited, and can be selected from commercially available products.

The proliferation method (producing method) of the recombinant expression vector is not particularly limited, and conventional methods can be used therefor. Generally, *Escherichia coli* are used as a host to proliferate the vector. Here, *Escherichia coli* may be suitably selected according to the type of vector used.

### [2-1-2] Transformation step

The transformation step according to the present invention is not particularly limited as long as the recombinant expression vector for expressing double stranded RNA corresponding to the entire part or some part of the nucleotide sequence of a specific gene is introduced into the lipid producing fungi. As such, the procedure, condition, or material used in the step is not particularly limited. That is, in the transformation step, the recombinant expression vector constructed in the expression vector constructing step is introduced into the lipid producing fungi (transformation).

By carrying out the transformation step, a large amount of double stranded RNA corresponding to the entire part or some part of a desired gene whose expression is to be suppressed can be expressed in the lipid producing fungi. In the resulting transformed lipid producing fungi, expression of the desired gene is efficiently suppressed by RNAi.

As described above, a gene whose expression is to be suppressed is a GLELO gene (GB accession No. AF206662), a MAELO gene (GB accession No. AF268031), a Δ5 fatty acid desaturase gene (GB accession No. AY464949, AF067654, AF054824), Δ6 fatty acid desaturase gene (GB accession No. AB070557, AB070556, AB070555, AF307940, AF110510, AB020032), or a Δ12 fatty acid desaturase gene (GB accession No. AF417244, AF110509, AB020033,

Eur. J. Biochem. 261, 812-820 (1999)). In addition to these also the expression of the following genes can be suppressed: a Δ8 fatty acid desaturase gene, a Δ9 fatty acid desaturase gene (GB accession No. AJ278339, AF085500, Y18554, Y18553, AB015612, AB015611), a Δ15 fatty acid desaturase gene, a Δ17 fatty acid desaturase gene, and a ω3 fatty acid desaturase gene. By suppressing expression of genes involved in lipid metabolism, the yield of desired lipids can be increased or decreased. Further, the type or composition of produced lipids can be modified.

In the Examples below, MAELO gene and Δ12 fatty acid desaturase gene are used as examples of genes involved in lipid metabolism.

The MAELO gene is known to exhibit a weak activity for generating dihomo-γ-linolenic acid (DGLA, 20:3n-6) via γ-linolenic acid (GLA, 18:0n-6) (Japanese PCT Laid-Open Publication No. 2002-523098). However, it is believed that a gene that plays a principal role in this reaction is the GLELO gene, which has been isolated. As such, other functions of the MAELO gene are suspected. According to an annotation of the GenBank, a role of the MAELO gene in the catalysis of the chain-elongating reaction of saturated fatty acids or monounsaturated fatty acids has been suggested. However, there has been no report that suggests functions of the MAELO gene in *Mortierella* (lipid producing fungi), and particularly in *M. alpina* (*Mortierella alpina*)*.* Accordingly, there is a need to analyze functions of the MAELO gene in *M. alpina.*

As will be described later in Examples, the inventors of the present invention analyzed functions of the MAELO gene in *M. alpina* using yeasts. It was found as a result that the MAELO gene is involved in the catalysis of the chain-elongating reaction of very long-chain saturated fatty acids. Further, by suppressing expression of the MAELO gene in *M. alpina* by the RNAi method, a strain with a reduced proportion of very long-chain saturated fatty acids in lipids was obtained. These results revealed, for the first time, that the MAELO gene is involved in the biosynthesis of very long-chain saturated fatty acids in *M. alpina.*

The Δ12 fatty acid desaturase gene encodes an enzyme that catalyzes the reaction of converting oleic acid (18:1) to linoleic acid (18:2). Thus, by suppressing expression of the Δ12 fatty acid desaturase gene by the RNAi method as in the Examples below, a strain (mutant strain) can be obtained in which a proportion of ω9 fatty acids, such as mead acid, is increased.

The present invention also discloses an application even when a gene whose expression is to be suppressed is not specified. For example, when only a nucleotide sequence of EST with unknown functions is known, an expression vector that encodes double stranded RNA corresponding to the EST is constructed. By obtaining transformant strains with the expression vector and analyzing functions of the transformed strains, functions of the gene with the EST can be analyzed. In this manner, with the disclosed breeding method, unknown functions of a gene can be predicted.

A transformation method (gene introducing method) used in the transformation step is not particularly limited, and conventional methods such as an electroporation method or particle delivery method can be suitably used. When using an electroporation method in *Mortierella,* it is preferable that the fungi be used in the form of protoplasts. A non-limiting example of the particle delivery method is a particle gun method. In the Examples below, a particle delivery method was used for the transformation.

### [2-2] Other steps, other methods

A breeding method according to the present invention may include steps other than the transformation step or recombinant vector constructing step. One specific example of such an additional step is a screening step for selecting a suitable transformant strain from a group of transformed lipid producing fungi.

A screening method is not particularly limited. For example, after culturing and growing transformant strains, a strain may be selected based on predetermined genetic characteristics. That is, a specific method of the screening step is not particularly limited. Depending on the purpose of breeding, such conditions are set that allow a transformant strain with desirable characteristics to be selected, and a conventional method is used to select such a transformant strain. For example, selection can be made using a conventional auxotrophic marker or drug resistant marker.

It should be noted here that a gist of the present invention is to provide a method for breeding a novel strain of desired characteristics (traits) by suppressing expression of a predetermined gene in *Mortierella* (lipid producing fungi). Accordingly, the present invention is not just limited to the individual procedures of transformation, culturing, and selection as specifically described above. Instead, the invention also includes a breeding method employing other procedures.

### [3] Use of the present invention

### [3-1] Lipid producing fungi (novel breed)

As described above, a breeding method of lipid producing fungi according to the present invention is adapted to suppress expression of one of the above specified genes and thereby provide a breed with improved characteristics (traits). Thus, with the method, a novel breed (novel strain) can be efficiently and effectively produced by using lipid producing fungi of genus *Mortierella* as an original breed. The *Mortierella* are well known lipid producing fungi, and include highly reliable species such as *M*. *alpina*. Thus, for example, a strain with improved lipid productivity can be produced both easily and efficiently.

Further, with a breeding method according to the present invention, expression of any gene in the transformed lipid producing fungi can be suppressed. Thus, by suppressing expression of a specific gene in the original breed *Mortierella,* the invention provides a transformant strain (transformant), i.e., a novel strain, capable of producing more lipids, and/or modifying the type or composition of the lipids it produces. That is, the present invention discloses lipid producing fungi produced by the breeding method.

Further, by obtaining suitable DNA fragments (cDNA fragments, EST, etc.) from the original breed *Mortierella,* and by causing RNAi using an expression vector that encodes double stranded RNA corresponding to the DNA fragments, the invention discloses, for example, a transformant strain in which expression of the total length gene of the DNA fragments is suppressed. Through a functional analysis of the transformant strain, unknown functions of the gene can be predicted. That is, the present invention also discloses a method of predicting unknown functions of a gene.

As described above, a transformant strain obtained by a breeding method of the present invention is highly useful. Also disclosed is a breeding kit for implementing the invention, as described below.

### [3-2] Breeding kit

A breeding kit is not particularly limited as long as it is for implementing the breeding method described in section [2] above. As such, specific structures, materials, devices, and the like included in the breeding kit are not particularly limited. Specifically, a breeding kit is adapted to carry out the respective steps of the breeding method.

For example, in order to carry out the transformation step, the breeding kit may include (a) a recombinant expression vector for expressing double stranded RNA corresponding to the entire part or some part of the nucleotide sequence of a specific gene. Further, in order to carry out the expression vector constructing step, the breeding method may include (b) reagents for constructing the recombinant expression vector (a). Further, in order to carry out the transformation step, the breeding kit may include (c) reagents for introducing the recombinant expression vector (a) into lipid producing fungi. Further, the breeding method may additionally include (d) reagents for culturing the lipid producing fungi and/or a transformant strain into which the recombinant expression vector (a) have been introduced.

As used herein, the term "reagents" include various types of reagents, experiment equipment, devices, or the like. For example, for the transformation, conventional reagents can be used. Specific forms of the reagents are not particularly limited. For example, enzymes or buffers can be used depending on the type of transformation required. Optionally, an experimental tool such as a micro centrifugal tube may be used. Further, competent cell-preparing drugs, or devices such as a heat block can be used as required. Further, in order to carry out the expression vector constructing step, various materials (carrier vector, restriction enzymes, etc.), various reagents, experimental equipment, detecting devices, etc. may be included.

With the breeding kit as exemplified above, a breeding method according to the present invention can be carried out both easily and reliably.

### [3-3] Producing method of lipid, and lipids obtained by the producing method

In a producing method of lipids according to the present invention, PUFA-containing lipids are produced from the lipid producing fungi described in section [3-1] above. For example, PUFA-containing lipids can be conveniently produced by culturing the lipid producing fungi.

In the following, lipids produced by *Mortierella* are described.

The PUFA-containing lipids are one of many useful products produced by *Mortierella.* A wild-type strain with good PUFA productivity is known to produce a large amount of ω6 PUFA that contains mostly arachidonic acid, and very long-chain saturated fatty acids (VLSA) are also produced as a byproduct. (Higashiyama K. et al. J. Am. Oil Chem. Soc., 75, 1501-1505, 1998). As used herein, the ω6 PUFA refers to those fatty acids containing 18 or greater carbon atoms with two or more double bonds, wherein the first double bond occurs at the sixth carbon from the carbon of the methyl terminus. Some of the representative examples of ω6 PUFA contained in *Mortierella* include linoleic acid, γ-linolenic acid, dihomo-γ-linolenic acid, and arachidonic acid.

Further, as used herein, "VLSA" refers to saturated fatty acids having 20 or greater carbon atoms with no double bond. Some of the representative examples of VLSA contained in *Mortierella* include eicosanoic acid (arachidic acid), docosanoic acid (behenic acid), tetracosanoic acid (lignoceric acid), and hexacosanoic acid (cerotic acid). Since these VLSAs are all biosynthesized from stearic acid, which is the precursor of arachidonic acid and other ω6 PUFAs, the VLSA biosynthetic pathway can be regarded as an alternative pathway of the ω6 PUFA biosynthetic pathway.

Generally, saturated fatty acids have considerably higher melting points than unsaturated fatty acids containing the same number of carbon atoms. For example, by comparing fatty acids containing 18 carbon atoms, oleic acid and linoleic acid, which are monounsaturated fatty acid and diunsaturated fatty acid, respectively, have melting points of 13.4°C and -5.2°C, which are significantly lower than the melting point 69.6°C of stearic acid, an example of saturated fatty acids. As to VLSA, these fatty acids have considerably high melting points, since the melting point increases with an increasing chain length. Docosanoic acid (behenic acid), tetracosanoic acid (lignoceric acid), hexacosanoic acid (cerotic acid) have melting points 79.9°C, 84.2°C, and 87.7°C, respectively. The melting point of lipids such as triglycerides is strongly influenced by the melting point of the constituting fatty acids, and therefore the melting point of oil or fat containing VLSA is generally high. For this reason, an oil or fat is often a solid at ordinary temperature, or may deposit and turn turbid or solidify when stored at a low temperature. For the separation and removal of a solid fat to obtain a liquid oil, procedures employing a wintering method or emulsion separation method are commonly used (Oils and Fats Handbook, SAIWAI SHOBO (1988) P. 261). An oil or fat produced by *Mortierella* contains several % VLSA, through the proportion varies depending on the culturing conditions.

For food use, it is often preferable that the oil or fat be stably provided as a transparent liquid even under low temperature conditions. While such lipids can be obtained by separating and removing a solid fat by the wintering method or emulsion separation method, the method involves complex procedures and therefore requires increased manufacturing time and increased cost.

Further, since the production of oil or fat favors the VLSA biosynthetic pathway which constitutes an alternative pathway of lipid biosynthesis, it is difficult to improve the yield of desired PUFA.

The ω6 PUFA containing mostly arachidonic acid constitutes most of the PUFA produced by a wild-type strain having good PUFA productivity. Mutation has created various types of mutant strains, and a large number of mutant strains producing modified PUFA are known. Analyses of these mutant strains have revealed that deactivating the Δ12 desaturase activity either completely or partially leads to accumulation of mead acid, and that complete or partial deactivation of the Δ5 desaturase activity leads to accumulation of dihomo-γ-linolenic acid.

In this manner, while different PUFAs can be produced by mutational modification, a mutant strain cannot be obtained without damaging genes of the PUFA-producing wild-type strain other than the genes associated with the target traits.

These problems can be solved by a method producing lipid producing fungi according to the present invention. That is, the method producing lipid producing fungi according to the present invention can reduce the amount of VLSA-containing lipids, which easily solidify to form fats. Therefore, with the method, liquid oil suitable for food can be produced without time-consuming and costly procedures such as the wintering method or emulsion separation method.

Further, because the said method does not favor the alternative VLSA biosynthesis pathway, a target PUFA can be produced with improved productivity. Further, a PUFA-producing strain can be obtained without damaging genes other than the gene associated with desired traits.

The present application also discloses lipids produced by the lipid producing method. It is preferable that the lipids contain ω9 PUFA in a proportion of not less than 8%, preferably not less than 8.8%, or more preferably not less than 9.4%, with respect to total fatty acids in the lipids.

Further, it is preferable that the lipids contain mead acid in a proportion of not less than 1.3%, or more preferably not less than 1.6%.

Further, it is preferable that the lipids contain arachidonic acid in a proportion of not less than 10% with respect to total fatty acids, and very long-chain saturated fatty acids in a proportion of not more than 0.1% with respect to total fatty acids. More preferably, the lipids contain arachidonic acid in a proportion of not less than 20% with respect to total fatty acids, and very long-chain saturated fatty acids in a proportion of not more than 0.1% with respect to total fatty acids.

That is, the present application also discloses the following lipids:
- Lipids produced by the lipid producing method, containing ω9 PUFA in a proportion of not less than 8% with respect to total fatty acids of the lipids.
- Lipids produced by the lipid producing method, containing mead acid in a proportion of not less than 1.3% with respect to total fatty acids of the lipids.
- Lipids produced by the lipid producing method, containing arachidonic acid in a proportion of not less than 10% with respect to total fatty acids of the lipids, and very long-chain saturated fatty acids in a proportion of not more than 0.1% with respect to total fatty acids of the lipids.

While the invention is susceptible to various modifications and alternative forms, a specific embodiment thereof will be described below in more detail by way of Examples. It should be understood, however, that it is not intended to limit the invention to the particular forms disclosed, but on the contrary, the invention is to cover all modifications, equivalents, and alternatives falling within the scope of the invention as defined in the appended claims.

### [Examples]

### (Example A) Functional analysis of MAELO gene

A plasmid for expressing MAELO gene in yeasts was prepared according to the following procedure.

First, *M. alpina* was cultured for 5 days in GY medium (2% glucose, 1% yeast extract, pH = 6.0), and total RNA was extracted using RNeasy plant mini kit (QIAGEN). Then, a reverse transcription reaction was carried for 1 µg of total RNA, using SuperScript First-Strand Synthesis System for RT-PCR (Invitrogen), so as to synthesize cDNA. For the reaction, oligo-dT primer was used. By using 1 µg of cDNA as a template, PCR was carried out with primer MAELO-H1, primer MAELO-S1, and ExTaq (TaKaRa). The PCR product was digested with restriction enzyme HindIII and restriction enzyme SpeI, and a DNA fragment of about 950 bp was ligated to vector pYES2 (Invitrogen) digested with restriction enzyme HindIII and restriction enzyme XbaI. As a result, plasmid pY2MEL was constructed.
MAELO-H1: 5'-gcaagcttatggccgccgcaatcttggac-3' (SEQ ID NO: 15)
MAELO-S1: 5'-gcactagtttagatgtgcttgctgttggag-3' (SEQ ID NO: 16)

The plasmid pY2MEL was used to transform a *S*. *cerevisiae* INVSc 1 strain, and strains that grew on a uracil-deficient plate (2% glucose, 0.17% Yeast Nitrogen Base w/o Amino Acids and Ammonium Sulfate (Difco), 0.5% ammonium sulfate, histidine (20 mg/l), leucine (60 mg/l), tryptophan (40 mg/l), 2% Bacto agar) were selected as transformed strains. These strains were then inoculated once with a platinum loop on a medium containing 2% raffinose, 0.17% Yeast Nitrogen Base w/o Amino Acids and Ammonium Sulfate (Difco), 0.5% ammonium sulfate, 1% Tergitol Type NP-40, histidine (20 mg/l), leucine (60 mg/l), tryptophan (40 mg/l), and 0.05% stearic acid (18:0), and the medium was cultured for 6 hours by shaking. Then, 2% (w/v) galactose was added to the medium so as to induce expression of MAELO gene ligated downstream of the GAL1 promoter in the plasmid pY2MEL, and the medium was cultured at 28°C for 42 hours by shaking. The fungi were collected by centrifugation, and freeze-dried. After inducing the fatty acid residue in the fungi to methyl ester by a hydrochloric acid/methanol method, the sample was extracted with hexane. The resulting fatty acid methyl ester after the removal of hexane was analyzed by gas chromatography. Table 1 below represents the yields of very long-chain saturated fatty acids in each broth.

**[Table 1]**

| | Yields of very long-chain saturated fatty acids (mg/l) | | | |
|---|---|---|---|---|
| | 20:0 | 22:0 | 24:0 | 26:0 |
| pYES2-introduced strain | 0.78 | 0.06 | 0.06 | 0.65 |
| pY2MEL-introduced strain | 0.63 | 0.12 | 0.34 | 1.4 |

As shown in Table 1, the pY2MEL-introduced strain produced more 22:0, 24:0, and 26:0 very long-chain saturated fatty acids as compared with the pYES2-introduced strain (control). The result suggests the chain elongating activity of the MAELO gene for very long-chain saturated fatty acids.

### (Example B) Breeding of MAELO gene expression suppressing strain

According to the following procedure, a plasmid for causing excess expression of double stranded RNA corresponding to a portion of MAELO gene was constructed.

Plasmid pBluescriptIISK+ was digested with restriction enzymes SpeI and BamHI, and the ends were blunted with the DNA blunting Kit (TaKaRa). By allowing for self-ligation, plasmid pBluescriptIISK+(BamHI-) was constructed. Then, by using plasmid pD4 (D. A. Mackenzie et al. Appl. Environ. Microbiol., 66, 4655-4664, 2000) as a template, PCR was carried out with LA Taq (TaKaRa). As the primers, primer HisProFX and primer TrpCRX were used. The reaction was carried out in 30 cycles at 94°C for 1 minute, 55°C for 1 minute, and 72°C for 2 minutes. The amplified DNA fragments were digested with restriction enzyme EcoRI, and the resulting DNA fragments were inserted at the EcoRI site of the plasmid pBluescriptIISK+(BamHI-), so as to construct plasmid pBlueHpt.
HisProFX: 5'-tacgaattcaagcgaaagagagattatgaa-3' (SEQ ID NO: 1)
TrpCRX: 5'-gaagaattccctctaaacaagtgtacctgt-3' (SEQ ID NO: 2)

*M. alpina* was cultured at 28°C for 5 days in GY broth (2% glucose, 1% yeast extract, pH = 6.0). From the resulting fungi, genomic DNA was prepared according to the procedure of E. Sakuradani et al. Eur J. Biochem., 260, 208-216, 1999.

By using the genomic DNA of *M. alpina* as a template, PCR was carried out with LA Taq (TaKaRa). As the primers, primer MAELORNAi1-1 and primer MAELORNAi3-1 were used. The reaction was carried out in 30 cycles at 94°C for 1 minute, 55°C for 1 minute, and 72°C for 1 minute. The amplified DNA fragment of about 0.9 kb was TA cloned into the pTBlueT-Vector (TaKaRa). After confirming the nucleotide sequence, the fragment was digested with restriction enzymes NcoI and BamHI. The resulting DNA fragment of about 0.9 kb was inserted in the NcoI-BamHI site of the plasmid pBlueHpt, so as to construct plasmid pBlueMEi3.
MAELORNAil-1: 5'-ctggatcctatggccgccgcaatcttggaca-3' (SEQ ID NO: 3)
MAELORNAi3-1: 5'-aaccatggtcatccctaggtggaagtaatg-3' (SEQ ID NO: 4)

By using the genomic DNA of *M. alpina* as a template, PCR was carried out with LA Taq (TaKaRa). As the primers, primer MAELORNAi 1 and primer MAELORNAi5 were used. The reaction was carried out in 30 cycles at 94°C for 1 minute, 55°C for 1 minute, and 72°C for 1 minute. The resulting DNA fragment of about 0.7 kb was TA cloned into the pTBlueT-Vector (TaKaRa). After confirming the nucleotide sequence, the fragment was digested with restriction enzymes NcoI and B1nI. The resulting DNA fragment of about 0.7 kb was inserted in the NcoI-BlnI site of the plasmid pBlueMEi3, so as to construct plasmid pBlueMEi5.
MAELORNAi1: 5'-ttggatccatggccgccgcaatcttggaca-3' (SEQ ID NO: 5)
MAERORNAi5: 5'-tgatctcctaggtggaacactgatagccac-3' (SEQ ID NO: 6)

A fragment of about 3.3 kb obtained by digesting the plasmid pBlueMEi5 with the restriction enzyme EcoRI was inserted in the EcoRI site of plasmid pDura5, so as to construct plasmid pDura5MEi51.

By using the genomic DNA of *M. alpina* as a template, PCR was carried out with LA Taq (TaKaRa). As the primers, primer MAELORNAi1-1 and primer MAELORNAi2 were used. The reaction was carried out in 30 cycles at 94°C for 1 minute, 55°C for 1 minute, and 72°C for 1 minute. The amplified DNA fragment of about 0.7 kb was then TA cloned into the pT7Blue T-Vector (TaKaRa). After confirming the nucleotide sequence, the fragment was digested with restriction enzymes NcoI and BamHI. The resulting DNA fragment of about 0.7 kb was then inserted in the NcoI-BamHI site of the plasmid pBlueHpt, so as to construct plasmid pBlueMEi2.
MAELORNAi2: 5'-tgccatggggaaatatgccctaggccatgc-3' (SEQ ID NO: 17)

By using the genomic DNA of *M. alpina* as a template, PCR was carried out with LA Taq (TaKaRa). As the primers, primer MAELORNAi1 and primer MAELORNAi4 were used. The reaction was carried out in 30 cycles at 94°C for 1 minute, 55°C for 1 minute, and 72°C for 1 minute. The amplified DNA fragment of about 0.5 kb was TA cloned into the pT7Blue T-Vector (TaKaRa). After confirming the nucleotide sequence, the fragment was digested with restriction enzymes NcoI and BInI. The resulting DNA fragment of about 0.5 kb was then inserted in the NcoI-BlnI site of the plasmid pBlueMEi2, so as to construct plasmid pBlueMEi4.
MAELORNAi4: 5'-cacttacctaggggcttcttcttgagg -3' (SEQ ID NO: 18)

A fragment of about 2.9 kb obtained by digesting the plasmid pBlueMEi4 with the restriction enzyme EcoRI was inserted in the EcoRI site of plasmid pDura5, so as to construct plasmid pDura5Mei41. The plasmid pDura5Mei51 allows for excess expression of double stranded RNA corresponding to about 700 bp of the MAELO gene. In the case of plasmid pDura5Mei41, double stranded RNA corresponding to about 500 bp of the MAELO gene can be expressed in excess.

Using the plasmid pDura5MEi51 or pDura5Mei41, transformation was carried out by a particle delivery method. As the host, *M. alpina* Δura-1 strain (Δura5) was used.

For the selection of transformed strains, SC agar medium was used (5.0 g of Yeast Nitrogen Base w/o Amino Acids and Ammonium Sulfate (Difco), 1.7 g of (NH₄)₂SO₄, 20 g of glucose, 20 mg of adenine, 30 mg of tyrosine, 1.0 g of methionine, 2.0 mg of arginine, 2.0 mg of histidine, 4.0 mg of lysine, 4.0 mg of tryptophan, 5.0 mg of threonine, 6.0 mg of isoleucine, 6.0 mg of leucine, 6.0 mg of phenylalanine, and agar (20 g/l)).

Several ten transformed strains obtained for each plasmid were inoculated on GY medium. For each plasmid used, two of the transformed strains which stably held the marker ura5 gene were selected. Specifically, transformed strains #1 and #2 were used for cultures transformed by pDura5Mei51, and transformed strains #3 and #4 were used for cultures transformed by pDura5Mei41. The transformed strains #1 and #2 are for expressing double stranded RNA corresponding to about 700 bp of MAELO gene. The transformed strains #3 and #4 are for expressing double stranded RNA corresponding to about 500 bp of MAELO gene. For each of the transformed strains #1 through #4, the presence or absence of the gene was confirmed by PCR. Specifically, genomic DNA was prepared from each transformed strain, and PCR was carried out with EX Taq (TaKaRa) using the genomic DNA as a template. As the primers, primer RDNA1 and primer RDNA2 were used. The reaction was carried out in 35 cycles at 94°C for 1 minute, 54°C for 1 minute, and 72°C for 1 minute. As a result, the presence of amplified DNA fragment of about 1.5 kb was confirmed in all of the transformed strains # 1 through #4, whereas no amplification of DNA fragment was observed in the Δura-1 strain.

It was therefore confirmed that the DNA fragment was introduced through recombination of pDura5MEi51 or pDura5Mei41 in the 18SrDNA region of the transformed strains #1 through #4.
Primer RDNA1: 5'-acaggtacacttgtttagag-3' (SEQ ID NO: 7)
Primer RDNA2: 5'-cgctgcgttcttcatcgatg-3' (SEQ ID NO: 8)

Each transformed strain was then inoculated in a test tube containing 10 ml of GY broth. The sample was cultured at 28°C for 12 days by shaking, and the fungi were collected by filtration.

From a removed portion of fungi, total RNA was extracted using the RNeasy plant mini kit (QIAGEN). Then, a reverse transcription reaction was carried for 1 µg of total RNA, using SuperScript First-Strand Synthesis System for RT-PCR (Invitrogen), so as to synthesize cDNA. For the reaction, a random hexamer was used as a primer. By using 1 µg of cDNA as a template, PCR was carried out with primer MAELO-1, primer MAELO-2, and ExTaq (TaKaRa). The reaction was carried out in 20 cycles at 94°C for 1 minute, 55°C for 1 minute, and 72°C for 1 minute.
Primer: MAELO-1: 5'-agtccatcgactccttcgtcttcca-3' (SEQ ID NO: 9)
Primer: MAELO-2: 5'-cggtgtcagccaactcccagtactt-3' (SEQ ID NO: 10)

The PCR product was electrophorased, and fluorescence intensities were compared between the bands of about 340 bp fragments. The result showed a distinct band in the Δura-1 strain, while no band was observed in any of the transformed strained #1 through #4. It was therefore confirmed that expression of mRNA of the MAELO gene was suppressed in the transformed strains #1 through #4. The remaining fungi were dried, and the fatty acid residue in the fungi was induced to methyl ester by a hydrochloric acid/methanol method. After extraction with hexane, the resulting fatty acid methyl ester was analyzed by gas chromatography. The results are shown in Table 2 below.

**[Table 2]**

| | Control | Transformed strains | | | |
|---|---|---|---|---|---|
| | Δura-1 | #1 | #2 | #3 | #4 |
| Proportions in total fatty acids (%) | | | | | |
| γ-linolenic acid | 4.2 | 4.9 | 4.3 | 3.7 | 3.3 |
| Dihomo-γ-linolenic acid | 3.0 | 2.8 | 2.7 | 3.0 | 2.9 |
| Arachidonic acid | 23.2 | 22.6 | 26.7 | 25.1 | 25.2 |
| 22:0 | 1.3 | 0.0 | 0.0. | 0.6 | 0.6 |
| 24:0 | 3.4 | 0.1 | 0.1 | 0.3 | 0.3 |
| 26:0 | 0.0 | 0.0 | 0.0 | 0.0 | 0.0 |

As can be seen from Table 2, the proportion of very long-chain saturated fatty acids dropped significantly in the transformed strains. Specifically, synthesis of very long-chain saturated fatty acids was completely suppressed in the transformed strains #1 and #2, whereas their synthesis was partially suppressed in the transformed strains #3 and #4.

### (Example C) Breeding of Δ12 fatty acid desaturase gene expression suppressing strain

For the excess expression of double stranded RNA corresponding to a portion of the Δ12 fatty acid desaturase gene, a vector was constructed according to the following procedure.

By using plasmid pMOD10 (Eur. J. Biochem. 261, 812-820 (1999)) as a template, PCR was carried out with primer Δ12-1, primer Δ12-2, and LA Taq (TaKaRa), so as to amplify a DNA fragment of about 670 bp. The reaction was carried out in 30 cycles at 94°C for 1 minute, 55°C for 1 minute, and 72°C for 1 minute.
Primer Δ12-1: 5'-gcggatccatggcacctcccaacacta-3' (SEQ ID NO: 11)
Primer Δ12-2: 5'-agaggccttcataataaggtacgcaggc-3' (SEQ ID NO: 12)

The amplified DNA fragment was digested with restriction enzymes BamHI and StuI, and was ligated to an about 3.7 kb fragment obtained by digesting the plasmid pMOD 10 with BamHI and MscI, using the ligation high (TOYOBO), so as to obtain plasmid pBΔ12RNAi. The plasmid pBΔ12RNAi was then digested with restriction enzyme EcoRI, and the ends were blunted with the DNA blunting kit (TaKaRa). By digesting with the restriction enzyme BamHI, a fragment of about 1.1 kb was obtained. The plasmid pBlueHpt was digested with restriction enzyme NcoI, and the ends were blunted with the DNA blunting kit (TaKaRa). By digesting with the restriction enzyme BamHI, a DNA fragment of about 4.7 kb was obtained. These two fragments were ligated to each other with the ligation high (TOYOBO), so as to obtain plasmid pBlueΔ12RNAi. The plasmid pBlueΔ12RNAi was digested with the restriction enzyme EcoRI, and the resulting DNA fragment of about 2.8 kb was inserted in the EcoRI site of plasmid pDura5, so as to obtain plasmid pDura5Δ12RNAi

The plasmid pDura5Δ12RNAi was used to transform the *M. alpina* Δura-1 strain, using a particle delivery method. For the selection of transformed strains, strains that can grow in SC agar medium were selected. Several ten transformed strains were inoculated on GY medium, and strains that stably held the marker ura5 gene were selected. As in Example B, it was confirmed that three of the selected strains (transformed strains #5 through #7) had incorporated the plasmid pDura5Δ12RNAi, through recombination, in the 18SrDNA region of the chromosome. Each transformed strain was cultured at 28°C for 5 days in a flask containing 500 ml of SC broth. For the breeding of the Δura-1 strain (control), uracil was added (50 mg/1) to the SC broth.

From a removed portion of the fungi, cDNA was synthesized according to the procedure of Example B, and PCR was carried out using primer Δ12-3, primer Δ12-4, and Ex Taq (TaKaRa). The reaction was carried out in 20 cycles at 94°C for 1 minute, 55°C for 1 minute, and 72°C for 1 minute.
Primer Δ12-3: 5'-ttgctattgatctgacctgggcctc-3' (SEQ ID NO: 13)
Primer Δ12-4: 5'-tgggaacaaagacctggtccttgg-3' (SEQ ID NO: 14)

The PCR product was electrophorased, and fluorescence intensities were compared between the bands of about 310 bp fragments. The result confirmed a distinct band in the Δura-1 strain, whereas no band was observed in any of the transformed strains #5 through #7. It was therefore confirmed that expression of mRNA of the Δ12 fatty acid desaturase gene was suppressed in the transformed strains #5 through #7.

The fatty acids produced by the fungi were analyzed as in Example B. The results are shown in Table 3 below.

**[Table 3]**

| | Control | Transformed strains | | |
|---|---|---|---|---|
| | Δura-1 | #5 | #6 | #7 |
| Proportions in total fatty acids (%) | | | | |
| ω6 PUFA | | | | |
| linoleic acid | 14.8 | 0.1 | 0.0 | 0.1 |
| γ-linolenic acid | 3.4 | 0.2 | 0.0 | 0.5 |
| Dihomo-γ-linolenic acid | 2.4 | 0.2 | 0.2 | 0.1 |
| Arachidonic acid | 11.9 | 1.5 | 1.1 | 2.3 |
| Total of ω6 fatty acids | 32.5 | 2.0 | 1.3 | 3.0 |
| ω9 PUFA | | | | |
| 18:2 ω9 | 0.2 | 6.3 | 6.9 | 5.9 |
| 20.2 ω9 | 0.0 | 0.9 | 0.9 | 0.8 |
| Mead acid | 0.0 | 1.6 | 1.6 | 1.3 |
| Total of ω9 fatty acids | 0.2 | 8.8 | 9.4 | 8.0 |

As can be seen from Table 3, the proportion of ω6 PUFA dropped in the transformed strains, but there was a significant increase in the proportion of ω9 PUFA. Specifically, while a considerable amount of mead acid was present in the transformed strains, the Δura-1 strain did not contain any mead acid. That is, it was found that the ability to produce mead acid and other ω9 PUFAs can be obtained by suppressing expression of the Δ12 fatty acid desaturase gene.

### INDUCTRIAL APPLICABILITY

With a breeding method of lipid producing fungi according to the present invention, a mutant strain of lipid producing fungi of genus *Mortierella* can be obtained, in which expression of an above-mentioned lipid metabolism gene is suppressed. That is, by using lipid producing fungi of genus *Mortierella* as an original breed, a novel breed (novel strain) with desired characteristics can be produced both efficiently and effectively.

Further, since the invention allows for self-cloning, expression of an above-mentioned lipid metabolism gene can be suppressed by carrying out an RNAi method using DNA fragments suitably obtained from the original breed *Mortierella.* The invention therefore also discloses a transformant strain (transformant), i.e., a novel strain, capable of producing more lipids and/or modifying the type or composition of the lipids it produces.

The *Mortierella* are well known lipid producing fungi, and include highly reliable species such as *M. alpina.* Thus, with the present invention, a strain with improved lipid productivity, modified lipid composition, and/or improved productivity of specific lipids can be produced both easily and efficiently.

As described, a breeding method of lipid producing fungi according to the present invention can be used to efficiently and effectively produce lipid producing fungi of genus *Mortierella.* The invention is therefore applicable to industries related to various kinds of fermentation techniques using *Mortierella,* in addition to food, pharmaceutical, and other industries using such fermentation techniques.

## Claims

1. A method for the production of lipid producing fungi that belong to genus *Mortierella*, said method comprising an expression suppressing step of suppressing expression of a lipid metabolism gene encoding GLELO, MAELO, Δ5 fatty acid desaturase, Δ6 fatty acid desaturase or Δ12 fatty acid desaturase in the lipid producing fungi, wherein said expression suppressing step includes an RNAi step of suppressing expression of the lipid metabolism gene by an RNAi method or a cosuppression step of suppressing expression of the lipid metabolism gene by a cosuppression method.

2. The method of claim 1, wherein said RNAi step includes a transformation step of introducing a recombinant expression vector into the lipid producing fungi, wherein the recombinant expression vector causes expression of double stranded RNA corresponding to all of or part of a nucleotide sequence of the lipid metabolism gene.

3. The method of claim 2, wherein said RNAi step further includes an expression vector constructing step of constructing the recombinant expression vector.

4. The method of claim 2 or 3, wherein the transformation step is carried out by an electroporation method or a particle delivery method.

5. The method of any one of claims 1 to 4, wherein the lipid producing fungi are *Mortierella alpina.*

## Patentansprüche

1. Verfahren zur Herstellung von Lipid produzierenden Pilzen, die zur Gattung *Mortierella* gehören, umfassend einen Expressionsunterdrückungsschritt des Unterdrückens der Expression eines Lipidstoffwechselgens, das GLELO, MAELO, Δ5-Fettsäuredesaturase, Δ6-Fettsäuredesaturase oder Δ12-Fettsäuredesaturase codiert, in den Lipid produzierenden Pilzen, wobei der Expressionsunterdrückungsschritt einen RNAi-Schritt des Unterdrückens der Expression des Lipidstoffwechselgens durch ein RNAi-Verfahren oder einen Co-Unterdrückungsschritt des Unterdrückens der Expression des Lipidstoffwechselgens durch ein Co-Unterdrückungsverfahren beinhaltet.

2. Verfahren gemäß Anspruch 1, wobei der RNAi-Schritt einen Transformationsschritt des Einbringens eines rekombinanten Expressionsvektors in die Lipid produzierenden Pilze beinhaltet, wobei der rekombinante Expressionsvektor die Expression von doppelsträngiger RNA bewirkt, die der gesamten oder Teilen der Nucleotidsequenz des Lipidstoffwechselgens entspricht.

3. Verfahren gemäß Anspruch 2, wobei der RNAi-Schritt weiter einen Expressionsvektorkonstruktionsschritt der Konstruktion des rekombinanten Expressionsvektors beinhaltet.

4. Verfahren gemäß Anspruch 2 oder 3, wobei der Transformationsschritt mittels eines Elektroporationsverfahrens oder eines Partikelzuführungsverfahrens durchgeführt wird.

5. Verfahren gemäß einem der Ansprüche 1 bis 4, wobei die Lipid produzierenden Pilze *Mortierella alpina* sind.

## Revendications

1. Procédé de production de champignons produisant des lipides appartenant au genre *Mortierella*, ledit procédé comprenant une étape de suppression de l'expression supprimant l'expression d'un gène du métabolisme des lipides codant la GLELO, la MAELO, la Δ5-désaturase des acides gras, la Δ6-désaturase des acides gras ou la Δ12-désaturase des acides gras dans les champignons produisant des lipides, dans lequel ladite étape de suppression de l'expression inclut une étape d'ARNi supprimant l'expression du gène du métabolisme des lipides au moyen d'une méthode d'ARNi ou une étape de cosuppression supprimant l'expression du gène du métabolisme des lipides au moyen d'une méthode de cosuppression.

2. Procédé selon la revendication 1, dans lequel ladite étape d'ARNi inclut une étape de transformation introduisant un vecteur d'expression recombinant dans les champignons produisant des lipides, dans lequel le vecteur d'expression recombinant est à l'origine de l'expression d'ARN double brin correspondant à la totalité ou une partie d'une séquence nucléotidique du gène du métabolisme des lipides.

3. Procédé selon la revendication 2, dans lequel ladite étape d'ARNi comprend en outre une étape de construction du vecteur d'expression comprenant la construction du vecteur d'expression recombinant.

4. Procédé selon la revendication 2 ou 3, dans lequel l'étape de transformation est réalisée au moyen d'une méthode d'électroporation ou d'une méthode de libération de particule.

5. Procédé selon l'une quelconque des revendications 1 à 4, dans lequel les champignons produisant des lipides sont des *Mortierella alpina*.
